# FASCICULE DE BREVET EUROPEEN

(11) **EP 1 831 160 B1**
(45) Date de publication et mention de la délivrance du brevet: **30.06.2010**
(21) Numéro de dépôt: 05850628.8
(22) Date de dépôt: 29.12.2005
(51) Int. Cl.: C07C 323/52, C07C 323/60, C07C 319/18, C07C 319/20, A23K 1/16

(54) **SYNTHESE ET APPLICATIONS DE L'ACIDE 2-OXO-4-METHYLTHIOBUTYRIQUE, SES SELS ET SES DERIVES**
SYNTHESE UND VERWENDUNGEN VON 2-OXO-4-METHYLTHIOBUTTERSÄURE, DEREN SALZE UND DEREN DERIVATE
SYNTHESIS AND USES OF 2-OXO-4-METHYLTHIOBUTYRIC ACID, ITS SALTS AND ITS DERIVATIVES

(30) Priorité: 30.12.2004 FR 0414084
(43) Date de publication de la demande: 12.09.2007
(73) Titulaire: Adisseo Ireland Limited, Dublin 1 (IE)
(72) Inventeur: REY, Patrick, F-69006 Lyon (FR); BLANCHARD, Gilbert, F-60330 Lagny le Sec (FR)
(74) Mandataire: Maureau, Philippe
(86) Numéro de dépôt international: PCT/FR2005/003300
(87) Numéro de publication internationale: WO 2006/072711

(56) Documents cités:
- WO-A-2004/012706
- FR-A- 2 317 919
- K.J. CLEMETSON: "Production of alpha-keto acids: 2. Imoobilized whole cells of Providencia sp. PCM 1298 containing L-amino caid oxidase" ENZ.MICROB.TECHN., vol. 4, no. 6, 1982, pages 409-413, XP002348996 cité dans la demande
- H. RAPOPORT: "Synthesis of deuterium labeled plant ethylene precursor" J. LABEL.COMPDS. RADIOPHARM., vol. 36, no. 5, 1995, pages 431-437, XP009055202
- KUAJIMA, I.: "Quaternary ammonium fluoride-catalyzed conjugate addition of thiols to C=C double bonds" SYNTHESIS, 1976, pages 602-604, XP002348998
- NICOLET B H: "The addition of mercaptans to certain double bonds" JOURNAL OF THE AMERICAN CHEMICAL SOCIETY, AMERICAN CHEMICAL SOCIETY, WASHINGTON, DC, US, vol. 57, no. 6, 7 juin 1935 (1935-06-07), pages 1098-1099, XP002081891 ISSN: 0002-7863
- PIOTR W.D. SCISLOWSKI: "Methionine transamination-metabolic function and subcellular compartmentation" MOL. CELL. BIOCHEMISTRY, no. 129, 1993, pages 39-45, XP009055185

## Description

L'invention concerne la préparation de l'acide 2-oxo-4-méthylthiobutyrique (ci-après désigné par KMB), de ses sels, de ses dérivés, et leurs utilisations, notamment en nutrition animale.

La méthionine est un acide aminé soufré essentiel qui intervient dans de nombreux processus métaboliques dont principalement :

la synthèse protéique : la méthionine est non seulement un composant des protéines, au même titre que les autres acides aminés, mais également l'acide aminé qui initie le processus de la synthèse protéique ce qui la rend d'autant plus indispensable ;
le métabolisme lipidique : la méthionine intervient dans la synthèse des constituants des lipoprotéines sériques, et par conséquent elle joue un rôle dans le transport des lipides dans le sang, leur utilisation et leur dépôt dans les tissus.

Chez la volaille, la méthionine est un acide aminé essentiel et doit être ajoutée à l'alimentation.

Chez les vaches laitières, elle est un aminoacide limitant à l'égard de la production de lait. En outre, la méthionine intervient favorablement sur la fertilité et la fonction hépatique des vaches.

Une production suffisante de lait, et plus globalement, un meilleur état général des vaches, sont donc conditionnés par un régime adapté en méthionine. La production de lait peut même être accrue en augmentant la teneur en méthionine du régime.

Cependant la forme libre de la méthionine est rapidement dégradée par la flore bactérienne du rumen des vaches, et seule une très faible fraction de la méthionine intègre la circulation sanguine.

Pour surmonter cet inconvénient, des solutions ont été élaborées, en substituant à la méthionine, une méthionine protégée, chimiquement ou par enrobage, ou une méthionine modifiée, qui présente une biodisponibilité sanguine en méthionine intéressante. Ainsi, sont notamment connus une méthionine protégée, la Smartamine^{®}, fabriquée et commercialisée par la Demanderesse, et deux analogues de la méthionine, l'acide 2-hydroxy-4-méthylthiobutyrique (HMB) et l'ester isopropylique de HMB, peu affectés par une dégradation au niveau du rumen.

Les auteurs de la présente invention ont découvert que l'acide 2-oxo-4-méthylthiobutyrique (KMB) constitue un analogue de substitution de la méthionine avantageux en présentant une biodisponibilité en méthionine élevée, ledit analogue étant susceptible d'être obtenu par une voie synthétique simple, qui peut être mise en oeuvre à l'échelle industrielle.

Selon K. Mosbach et al., Enzyme and Microbial Technology (1982) 4, N°6, 409-413 et K.J. Clemetson et al., Toxicon (2002) 40, 659-665, l'acide 2-oxo-4-méthylthiobutyrique est préparé, à l'échelle du laboratoire, par une voie de synthèse enzymatique. Cette synthèse n'est toutefois pas adaptable à la production industrielle dudit acide, en raison des inconvénients inhérents aux procédés de fermentation, comme la complexité des infrastructures nécessaires à leur mise en oeuvre, les risques de pollution microbiologique, les durées élevées de réaction et les pertes de rendement imputables à l'énantiosélectivité du microorganisme sélectionné (H. Simon et al., Tetrahedron (1990) 47, N°43, 9019-9034).

H. Rapoport, J. Label. Compds. Radiopharm. (1994) Vol. 36, N°5, p431-437, décrit la synthèse de l'acide 2-oxo-4-méthylthiobutyrique en six étapes à partir de chlorure éthyl oxylyle. Dans cette synthèse, le méthylmercaptan est additionné sur l'éthyl-4-chloro-3-oxo-3-butènoate, avant ou après son hydrogénation.

Les auteurs ont élaboré un procédé de préparation de l'acide précité en deux étapes et dans des conditions déterminées permettant de limiter les temps de réaction et améliorant au surplus la sélectivité de réaction.

Ainsi, un premier objet de l'invention est un procédé pour préparer l'acide 2-oxo-4-méthylthiobutyrique répondant à la formule (I) suivante : 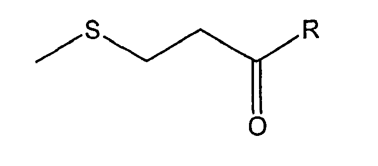 dans laquelle R représente un groupement carboxylique, et ses sels, ledit procédé comprenant les étapes suivantes :
on effectue l'oxydation catalytique et sélective du but-3-ène-1,2-diol (II) en acide-2-oxo-but-3-ènoïque (III), selon le schéma réactionnel (i) suivant : 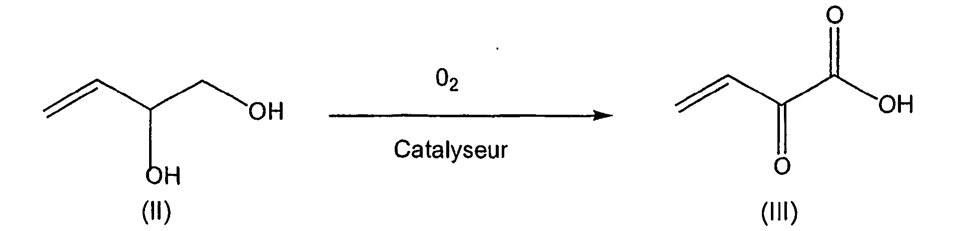
et on condense sélectivement le méthylmercaptan sur l'acide-2-oxo-but-3-ènoïque (III) selon le schéma réactionnel (ii) suivant :

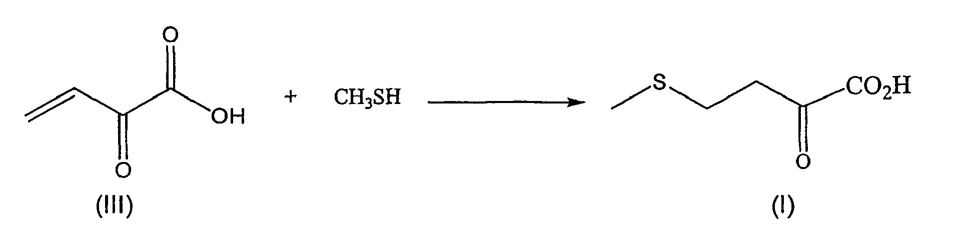

Ce procédé permet aussi de préparer des dérivés de l'acide 2-oxo-4-méthylthiobutyrique et les sels de ces dérivés, lesdits dérivés répondant à la formule (I) suivante : 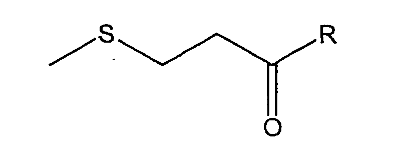 dans laquelle R représente un groupement choisi parmi COOR', CONH₂, CONHR', CONR'R" où R' et R" sont choisis, indépendamment l'un de l'autre, dans le groupe des radicaux alkyles linéaires, comme par exemple, les radicaux méthyle, éthyle, n-propyle, n-butyle et n-pentyle, des radicaux alkyles ramifiés, comme par exemple, les radicaux isopropyle, isobutyle, sec-butyle, tert-butyle, isopentyle et isohexyle, lesdits radicaux alkyles ayant de 1 à 12 atomes de carbone, et des radicaux cycloalkyles ayant de 3 à 12 atomes de carbone, procédé selon lequel on complète la préparation de l'acide 2-oxo-4-méthylthiobutyrique (I) ci-dessus, avec une étape d'estérification ou d'amidification, que l'homme du métier peut mettre en oeuvre en ayant recours à ces connaissances générales.

Selon l'invention, on entend par sels de l'acide 2-oxo-4-méthylthiobutyrique et de ces dérivés, les sels tels que ceux de calcium, sodium, magnésium, manganèse, zinc.

Des variantes et mises en oeuvre préférentielles du procédé de l'invention sont ci-après exposées, et, dans le cadre de la présente invention, leurs caractéristiques peuvent être considérées seules ou en combinaison.

Pour l'étape d'oxydation catalytique du but-3-ène-1,2-diol (II) en acide-2-oxo-but-3-ènoïque (III), le catalyseur répond avantageusement aux caractéristiques suivantes : Ledit catalyseur comprend au moins un métal noble choisi parmi le palladium, le platine, le ruthénium, l'iridium, le rhodium et leurs mélanges.

Le catalyseur à base de métaux nobles comprend au moins un promoteur choisi parmi le bismuth, le plomb, l'antimoine, l'étain, le niobium, le tellure, l'indium, le gallium, le zinc, le cuivre, le nickel, le cobalt, l'or, l'argent, le tungstène, le molybdène, le rhénium, le vanadium, le chrome, le manganèse, le fer et leurs mélanges.

La teneur du métal ou des métaux nobles est comprise entre 0,1 et 10% en poids par rapport au support catalytique, et de préférence, comprise entre 0,5 et 5% en poids.

Le catalyseur comprend en outre un support inerte choisi parmi l'alumine, la silice, les charbons actifs, le graphite, l'oxyde de titane, le zircone, le carbure de silicium, les oxydes mixtes à base de zirconium et de cérium, le noir d'acétylène.

La teneur en promoteur est comprise entre 0,005 et 500%, de préférence entre 0,005 et 100%, en poids du poids du métal ou des métaux nobles et/ou elle peut atteindre 10% en poids du poids du catalyseur. Le dépôt du promoteur sur le catalyseur à base de métaux nobles est avantageusement réalisé par imprégnation de ce promoteur sur le support catalytique.

Un catalyseur préféré comprend un métal noble choisi parmi le palladium, le platine et leurs mélanges, un promoteur choisi parmi le bismuth, le plomb et leurs mélanges et un support choisi parmi le charbon actif et le graphite.

Les conditions de la réaction d'oxydation sont avantageusement les suivantes : elle est réalisée en milieu alcalin ou neutre, à un pH maintenu entre 4 et 11, de préférence compris entre 5,5 et 7,5. A cet effet, on ajoute un agent alcalin choisi parmi l'hydroxyde de calcium, l'hydroxyde de sodium, l'hydroxyde de potassium, l'hydroxyde de lithium, l'hydroxyde de magnésium, l'ammoniac, le carbonate de sodium, le carbonate de zinc, le carbonate de manganèse et leurs mélanges. Elle est réalisée à une température comprise entre 10 et 95°C, de préférence entre 20 et 95°C, et mieux encore entre 25 et 70°C ; la durée de l'oxydation est généralement comprise entre 20 minutes et 15 heures.

L'étape d'oxydation peut être amorcée par l'apport d'un balayage d'un mélange gazeux contenant de l'oxygène, par exemple de l'air.

Les conditions de la réaction de condensation du méthylmercaptan sur l'acide-2-oxo-but-3-ènoïque sont avantageusement les suivantes : on utilise le méthylmercaptan sous forme gazeuse ou sous forme liquide ; elle est effectuée en présence d'un catalyseur basique. Le catalyseur est choisi parmi les amines aliphatiques telles que la méthylamine, l'éthylamine, la propylamine, la butylamine, la pentylamine, l'hexylamine, l'heptylamine et l'octylamine, les amines aromatiques comme l'aniline, la pyridine, l'hexaméthylènetétramine, la triéthylamine, la diisopropyléthylamine, diazabicylo[2,2,2]octane, la N,N-diméthylbenzylamine, la N-méthyldiphénylamine, la N-éthyl-3,3'-diphényldipropylamine, une N-alkylmorpholine, telle que la N-méthylmorpholine, ou le triton B. Ces amines organiques étant éventuellement, voire avantageusement, combinées à un acide organique ou minéral ; l'acide organique est de préférence choisi parmi l'acide formique, l'acide acétique, l'acide propanoïque et l'acide butanoïque et l'acide minéral l'est avantageusement parmi l'acide phosphorique et l'acide sulfurique.

Un autre objet de l'invention est le procédé pour préparer l'acide-2-oxo-but-3-ènoïque (III) et ses sels, notamment en tant que composé intermédiaire de la synthèse de l'acide 2-oxo-4-méthylthiobutyrique, selon lequel on effectue l'oxydation catalytique et sélective du but-3-ène-1,2-diol (II), selon le schéma réactionnel (i) dans l'une quelconque des conditions précitées, considérées seules ou en combinaison.

Comme dit précédemment, l'acide 2-oxo-4-méthylthiobutyrique et ses sels constituent des analogues de la méthionine, possédant une biodisponibilité élevée chez les vaches et les volailles. Ainsi, il est décrit un complément alimentaire consistant en un composé et/ou ses sels, ledit composé répondant à la formule (I) suivante : 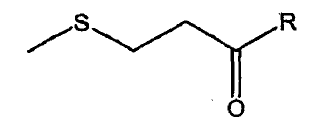 dans laquelle R représente un groupement choisi parmi COOR', CONH₂, CONHR', CONHR'R" où R' et R" sont choisis, indépendamment l'un de l'autre, dans le groupe des radicaux alkyles linéaires, comme par exemple, les radicaux méthyle, éthyle, n-propyle, n-butyle, n-pentyle, des radicaux alkyles ramifiés, comme par exemple, les radicaux isopropyle, isobutyle, sec-butyle, tert-butyle, isopentyle, isohexyle, ledits radicaux alkyles ayant de 1 à 12 atomes de carbone, et des radicaux cycloalkyles ayant de 3 à 12 atomes de carbone. De préférence, le complément de l'invention consiste en l'acide 2-oxo-4-méthylthiobutyrique (I) et/ou ses sels.

Il est aussi décrit une ration alimentaire comprenant une partie de céréales, une partie d'aliment concentré et un complément de l'invention tel que défini précédemment.

D'autres utilisations de l'invention sont un procédé d'administration de méthionine biodisponible à une vache, comprenant l'administration à la vache d'un complément ci-dessus, ainsi que l'utilisation, comme complément alimentaire pour la nutrition animale, d'un composé et/ou de ses sels, ledit composé répondant à la formule (I) 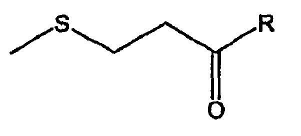 dans laquelle R représente un groupement choisi parmi COOR', CONH₂, CONHR', CONR'R" où R' et R" sont choisis, indépendamment l'un de l'autre, dans le groupe des radicaux alkyles linéaires, comme par exemple, les radicaux méthyle, éthyle, n-propyle, n-butyle, n-pentyte, les radicaux alkyles ramifiés, comme par exemple, les radicaux isopropyle, isobutyle, sec-butyle, tert-butyle, isopentyle, isohexyle, lesdits radicaux alkyles ayant de 1 à 12 atomes de carbone, et des radicaux cycloalkyles ayant de 3 à 12 atomes de carbone.

La présente invention est ci-après exposée plus en détails, puis illustrée à partir d'exemples mettant en évidence ses avantages.

### 1) Etape d'oxydation (i) :

### 1.a) Obtention du but-3-ène-1,2-diol (II) :

Le diol (II) peut être obtenu à partir du butadiène (IV) par monoépoxydation de celui-ci en 3,4-époxy-1-butène (V) qui est converti en diol (II) par ouverture chimique de la fonction époxyde.

Cette préparation est illustrée par les schémas réactionnels (iii) et (iv) ci-après :

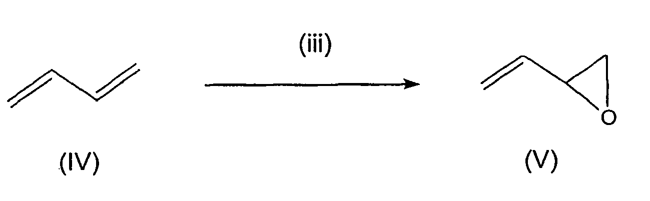

La réaction de monoépoxydation s'effectue en catalyse hétérogène à partir de catalyseurs supportés à base d'argent et activés par un promoteur choisi parmi les métaux alcalins tels que le potassium, le césium et le rubidium, comme décrit par exemple dans le document US-5 081 096 ou activés par le thallium tel que décrit dans le document US-5 138 077. Ces catalyseurs sont préparés par des méthodes conventionnelles telles que l'imprégnation et la co-précipitation.

La sélectivité de cette réaction en 3,4-époxy-1-butène (V), dans les conditions ci-dessus, peut excéder 95% et le rendement de conversion de (iii) oscille entre 12 et 15%. Au stade industriel, le butadiène (iv) non converti peut être recyclé au moins partiellement.

L'ouverture chimique de la fonction époxyde illustrée ci-après s'effectue classiquement en milieu aqueux :

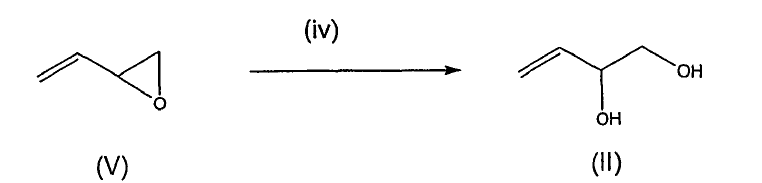

Cette ouverture est avantageusement acido-catalysée, en présence par exemple de résines acides dans des conditions notamment décrites dans les documents WO-A-91/15471 ou WO-A-00/24702.

### 1.b) Oxydation du but-3-ène-1,2-diol (II) :

Le diol (II) peut se présenter sous une forme liquide, à l'état purifié ou non, ou bien sous une forme de solution aqueuse brute, c'est-à-dire de moindre pureté, par exemple résultant de l'étape 1.a) ci-dessus.

Quelle que soit sa forme, le diol est directement utilisable pour la réaction d'oxydation catalytique en α-céto-acide (III). De manière avantageuse, la solution aqueuse qui résulte de l'ouverture de l'époxyde (V) sera directement engagée à l'étape d'oxydation du diol (II).

Le catalyseur d'oxydation du diol (II) comprend au moins un métal noble choisi parmi le palladium, le platine, le ruthénium, l'iridium, le rhodium et leurs mélanges. La teneur du métal ou des métaux nobles est comprise entre 0,1 et 10% en poids par rapport au support catalytique, et de préférence, comprise entre 0,5 et 5% poids.

Le support catalytique est choisi parmi l'alumine, la silice, les charbons actifs, le graphite, l'oxyde de titane, le zircone, le carbure de silicium, les oxydes mixtes à base de zirconium et de cérium, le noir d'acétylène.

Le catalyseur d'oxydation du diol (II) à base de métaux nobles comprend au moins un promoteur choisi parmi le bismuth, le plomb, l'antimoine, l'étain, le niobium, le tellure, l'indium, le gallium, le zinc, le cuivre, le nickel, le cobalt, l'or, l'argent, le tungstène, le molybdène, le rhénium, le vanadium, le chrome, le manganèse, le fer et leurs mélanges.

La teneur en promoteur est comprise entre 0,005 et 500%, de préférence entre 0,005 et 100%, en poids du poids du métal ou des métaux nobles. Le dépôt du promoteur sur le support catalytique est avantageusement réalisé par imprégnation.

Un catalyseur préféré comprend un ou des métaux nobles choisis parmi le palladium, le platine et leurs mélanges qui sont activés par du bismuth et/ou du plomb supportés sur du charbon actif ou du graphite.

La préparation du catalyseur est réalisée par imprégnation en maintenant sous agitation le mélange support de catalyseur et la solution contenant les métaux nobles, pendant une durée variant d'au moins quelques secondes à quelques heures, généralement comprise entre 15 minutes et 2 heures. Le catalyseur à base de métaux nobles est ensuite séché puis imprégné par la solution du promoteur. Cette opération précède l'étape de réduction du catalyseur qui est effectuée à une température comprise entre 20 et 400°C, à l'aide d'agents chimiques réducteurs du type formol, formiate de sodium, borohydrure de sodium, hydrogène, acide hypophosphoreux, hydrazine, glucose ou autres sucres réducteurs.

Une alternative de préparation du catalyseur est de réaliser une première imprégnation du promoteur suivie d'une seconde étape d'imprégnation du ou des métaux nobles. Une réduction du catalyseur est ensuite opérée.

Une autre alternative de préparation du catalyseur est de réaliser en une seule imprégnation du ou des métaux nobles et du promoteur. Une réduction du catalyseur est ensuite opérée.

Les détails du protocole opératoire de l'étape d'oxydation selon la présente invention sont ci-après exposés et seront illustrés dans les exemples :
- on introduit dans un réacteur muni d'un dispositif d'agitation, une solution aqueuse du diol (II), la concentration en diol (II) étant de préférence comprise entre 1 et 70% en poids. La limite inférieure de la concentration en diol est dictée par un souci de rentabilité du procédé et sa limite supérieure tient compte de la solubilité de l'oxygène dans les milieux considérés et du risque de cristallisation du sel de l'acide (III) formé pendant la réaction ;
- on disperse dans cette solution une quantité d'un catalyseur supporté et activé tel que décrit ci-dessus ;
- on amorce la réaction d'oxydation par l'apport simultané d'un balayage d'un gaz contenant de l'oxygène comme l'air. Le pH du milieu est régulé par l'ajout d'un agent alcalin. La température de réaction se situant généralement entre 10°C et 95°C, de préférence entre 20 et 95°C, voire entre 25°C et 70°C, pour un temps de réaction compris entre 20 minutes et 15 heures.

L'agent alcalin utilisé est avantageusement choisi parmi l'hydroxyde de calcium, l'hydroxyde de sodium, l'hydroxyde de potassium, l'hydroxyde de lithium, l'hydroxyde de magnésium, l'ammoniac, le carbonate de sodium, le carbonate de zinc, le carbonate de manganèse et leurs mélanges en fonction du but recherché. Il est également concevable d'utiliser du carbonate de zinc ou de manganèse ou tout autre sel de zinc ou de manganèse pour lesquels les hydroxydes correspondants sont obtenus *in situ* par addition d'un agent alcalin tel que la soude ou la potasse. L'agent alcalin est également destiné à neutraliser l'acide (III) produit, afin de maintenir une activité catalytique constante. L'agent alcalin doit effectivement maintenir le pH à une valeur suffisante pour assurer la désorption de l'acide (III) formé. Cette précaution permet, en outre, de s'affranchir de la formation de produits secondaires indésirables, par exemple issus d'une suroxydation du diol.

Dans la pratique, le pH est maintenu à une valeur comprise entre 4 et 11, de préférence entre 5,5 et 7,5. Le procédé selon l'invention permet d'atteindre des sélectivités très intéressantes excédant 90%. Ces performances ne sont pas altérées par un nombre important de recyclage et/ou de réactivation du catalyseur d'oxydation mis en oeuvre conformément à la présente invention. Les catalyseurs employés possèdent en effet une durée de vie appréciable et sont facilement régénérés *in situ* par dépôt d'une nouvelle charge de promoteur ou par réduction *in situ* du catalyseur désactivé.

Cette première étape d'oxydation est, avantageusement, pratiquée en solvant aqueux. Un solvant organique ou un mélange de solvants organiques peuvent aussi être employés. Un milieu hydro-organique peut aussi s'avérer profitable. Le solvant organique, constituant le milieu dans lequel s'effectue la réaction d'oxydation du diol (II), est choisi parmi tout solvant au moins partiel dudit diol (II), inerte dans les conditions opératoires. Les solvants sont choisis parmi les hydrocarbures aliphatiques, cycloaliphatiques ou aromatiques ; les esters d'alkyle ou alkényle d'acides carboxyliques aliphatiques ; les éthers aliphatiques, aromatiques ou cycliques ; les nitriles aliphatiques ; cycloaliphatiques ou aromatiques ; les cétones aliphatiques, cycloaliphatiques ou aromatiques. A titre d'exemples non limitatifs, on peut citer :
- des hydrocarbures comme le n-hexane, n-heptane, n-octane, n-nonane, le benzène, le styrène, l'éthylbenzène, le toluène, le métaxylène, l'isopropylbenzène, le cyclohexane, le méthyl-4-pentène-2 ;
- des esters comme le formiate d'éthyle, le formiate de butyle, le formiate d'isobutyle, l'acétate d'éthyle, l'acétate d'allyle, l'acétate de propyle, l'acétate de butyle, l'acétate d'hexyle, le propionate d'éthyle, le propionate de vinyle, l'acrylate d'éthyle, le butyrate de butyle, l'isobutyrate de méthyle ; le butyrate de méthyle ;
- des éthers comme l'éthoxy-1-butène-1 cis, l'éthoxy-1-butène-1-trans, l'oxyde de dibutyle, l'isopropoxy-1-butane, le diméthoxy-1,1-éthane, diéthoxy-1,1-éthane, diméthoxy-1,1-propane, l'éthoxy-1-butane, l'oxyde de diisopropyle, l'éthoxy-1-hexane, l'éthoxy-2-propane, le méthoxy-1-butadiène-1,3, l'oxyde de butyle et de vinyle, le furane, le diméthyl-2,5-furane ;
- des nitriles comme le butyronitrile, l'acétonitrile, l'acrylonitrile, le propionitrile, le tétrahydrobenzonitrile ;
- des cétones telles que la cyclopentanone, la dipropylcétone, l'heptanone, la méthylisopropylcétone, la méthyl-5-hexanone-2, la pentanone-2, la méthyl-4 pentène-3-one-2.

L'oxygène utilisé pour amorcer la réaction d'oxydation peut être de l'oxygène moléculaire, de l'air, de l'air enrichi, ou appauvri en oxygène ou tout autre mélange de l'oxygène avec un gaz inerte.

La pression totale sous laquelle s'effectue la réaction peut être supérieure, égale ou inférieure à la pression atmosphérique ; elle est généralement comprise entre 0,5 et 5 bars. La pression partielle d'oxygène est de préférence, comprise entre 0,05 bar et 2 bars. L'oxydation du diol (II) en α-céto-acide (III) peut être effectuée soit en maintenant une pression constante, soit en faisant circuler l'oxygène ou le gaz en contenant, dans l'appareil où s'effectue la réaction, soit encore en faisant barboter l'oxygène ou le gaz en contenant, dans le mélange réactionnel.

L'appareillage dans lequel le procédé selon l'invention est mis en oeuvre peut bien entendu ne pas être spécifique audit procédé.

### 2) Etape de condensation (ii) :

Selon cette étape, une mole de méthylmercaptan (MeSH) sous sa forme gazeuse ou liquide et une mole de l'α-céto-acide (III) précédemment préparé sont condensés selon le schéma réactionnel (ii) :

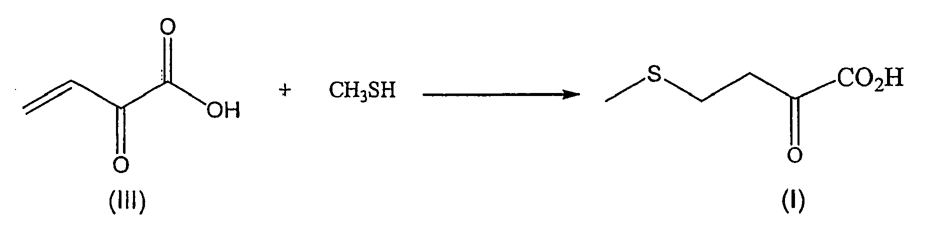

Le domaine de la présente invention est celui de la fabrication de l'acide (I) en tant que produit final, ou produit intermédiaire. La réactivité des thiols est, à bien des égards, similaire à celle des alcools. Ils peuvent suivant les conditions catalytiques mises en oeuvre s'additionner sur les aldéhydes α,β-insaturés, les cétones α,β-insaturés et les acides α,β-insaturés, en position 1,2 conduisant au mono-hémithioacétal ou en position 1,4 conduisant à l'aldéhyde 3-alkylthiopropionique. Par analogie structurale, l'acide (III) s'inscrit pleinement dans la catégorie des oléfines activées.

Deux voies catalytiques sont traditionnellement préconisées pour additionner sélectivement et efficacement les thiols en position 4 sur les dérivés carbonylés α,β-insaturés. La première est une addition ionique catalysée par les bases. La seconde est une addition radicalaire initiée par les composés azoïques ou peroxydes. Cependant, ce mode d'initiation conduit généralement à des polymères le plus souvent indésirables.

L'art antérieur détaille divers catalyseurs permettant d'orienter la régiosélectivité de l'addition de façon 1,2 ou 1,4. Néanmoins, l'addition 1,4 de type Michael des thiols sur les cétones α-β-insaturées reste la plus fréquente.

Le produit brut de départ comprenant l'acide (III) salifié ou non subit, éventuellement, un premier traitement permettant d'éliminer les impuretés coproduites lors de l'oxydation du diol (II). Ce produit brut peut aussi être soumis à un dégazage. L'excès du diol (II) correspondant au diol qui n'a pas réagi, peut être avantageusement recyclé à l'étape d'oxydation, par exemple, par distillation ou extraction. La solution aqueuse de l'acide (III) peut, éventuellement, subir une concentration préalable à la mise en contact avec le méthylmercaptan gazeux ou liquide. Cette solution aqueuse de l'acide (III) est ensuite mise en contact avec du méthylmercaptan gazeux ou liquide pour conduire à l'acide (I).

Cette étape peut éventuellement, être effectuée en présence d'un catalyseur ou d'un mélange de catalyseurs basiques. Des catalyseurs basiques appropriés sont, par exemple, les amines aliphatiques telles que la méthylamine, l'éthylamine, la propylamine, la butylamine, la pentylamine, l'hexylamine, l'heptylamine, l'octylamine, l'isopropylamine ; les amines aromatiques comme l'aniline, la benzylamine, la pyridine ; l'hexaméthylènetétramine, la triéthylamine, la diisopropyléthylamine, diazabicylo[2,2,2]octane, la N,N-diméthylbenzylamine, la N-méthyldiphénylamine, la N-éthyl-3,3'-diphényldipropylamine, une N-alkylmorpholine, telle que la N-méthylmorpholine, ou bien le triton B. Ces amines étant éventuellement combinées à un acide organique ou minéral ; celui-ci est de préférence choisi parmi l'acide formique, l'acide acétique, l'acide propanoïque et l'acide butanoïque, l'acide phosphorique et l'acide sulfurique.

L'addition du méthylmercaptan sur le α-céto-acide (III) est avantageusement acido-baso catalysée, par exemple au moyen d'un catalyseur consistant en une combinaison d'un acide organique ou minéral et une base organique ou minérale. L'acide acétique est préférentiellement utilisé.

A l'échelle industrielle, le méthylmercaptan, liquide ou gazeux, est acheminé dans un réacteur contenant la solution aqueuse, préalablement concentrée ou pas, dégazée ou non, de l'acide (III).

La condensation entre l'acide (III) et le méthylmercaptan peut être conduite en batch ou en continu. L'acide (III) et le méthylmercaptan sont introduits simultanément ou alternativement, en respectant le rapport stoechiométrique. On peut cependant envisager de travailler en défaut ou excès de méthylmercaptan, selon la réaction poursuivie.

La réaction peut se pratiquer par introduction continue entre la solution aqueuse de l'acide (III) et le méthylmercaptan gazeux dans un réacteur gaz/liquide. Dans ce cas, le méthylmercaptan pourra être ajouté à co- ou contre-courant. Alternativement, la réaction peut se pratiquer par introduction continue de la solution aqueuse de l'acide (III) et du méthylmercaptan liquide dans un réacteur batch ou piston. La température de réaction ne devra pas excéder 80°C.

Les catalyseurs de condensation entre l'acide (III) et le méthylmercaptan sont généralement choisis en fonction de plusieurs critères :
- la conversion et rendement en acide (I) ;
- la cinétique réactionnelle ;
- la sélectivité et tendance à coproduire des impuretés indésirables qui sont, habituellement, des espèces de haut poids moléculaire, issues de polymérisations parasites pendant la synthèse mais aussi durant le stockage du produit recherché ;
- la faculté à stabiliser le produit durant son stockage prolongé.

L'appareillage dans lequel le procédé selon l'invention est mis en oeuvre n'est pas spécifique audit procédé.

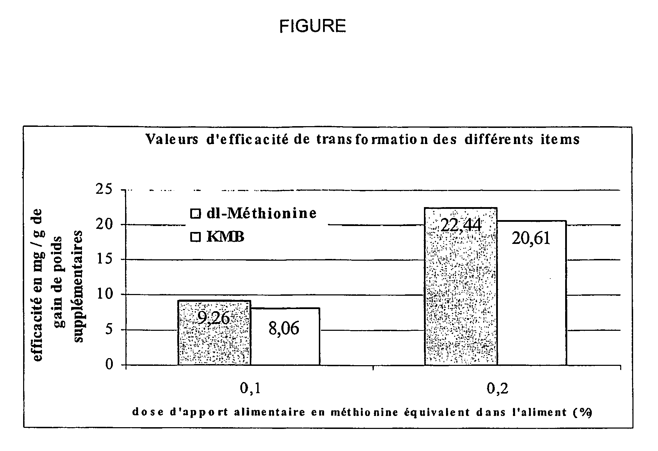

Les exemples qui suivent ont pour but d'illustrer l'invention sans en limiter la portée. Les exemples 1-7 illustrent la synthèse de composés de l'invention et les exemples 8 et 9 illustrent l'intérêt nutritionnel de composés de l'invention, à l'appui de la figure qui représente la valeur d'efficacité de la dl-Méthionine et du KMB en fonction de la dose d'application dans les essais de l'exemple 9.

### EXEMPLE 1: Préparation d'un catalyseur (A) : 1%Bi/5%Pt sur alumine

100 grammes de billes d'alumine de structure gamma sont préparées selon le procédé décrit dans la demande de brevet français FR-A-1 449 904, par autoclavage en présence d'acide, d'agglomérés d'alumine active, puis séchage et calcination. Ces billes présentent une surface spécifique de 100 m²/g, un volume poreux total de 0,90 cm³/g constitué par des macropores ayant un diamètre supérieur à 100 nm.

Ces billes sont ensuite imprégnées par 90 cm³ d'une solution de nitrate de bismuth contenant 1 gramme de bismuth.

Après 30 minutes de contact, les billes sont séchées à 150°C puis calcinées sous air à 600°C pendant 3 heures.

Elles sont ensuite imprégnées par 90 cm³ d'une solution d'acide chloroplatinique contenant 5 grammes de platine.

Après 30 minutes de contact, les billes sont séchées à 150°C puis activées pendant 3 heures à 300°C dans un courant d'hydrogène circulant à 200 litres par heure.

Le catalyseur (A) ainsi préparé contient en poids rapporté au support d'alumine 5 % de platine et 1 % de bismuth.

### EXEMPLE 2: Préparation d'un catalyseur (B) : 5%Bi/5%Pt sur charbon actif

100 grammes de charbon actif commercialisé par la société CECA sous l'appellation CECA 3S sont lavés successivement par une solution d'acide chlorhydrique puis par de l'eau permutée pour éliminer les impuretés solubles. Le support est ensuite séché à l'étuve à 120°C pendant 24 heures.

Ces pastilles sont ensuite imprégnées par une solution de nitrate de bismuth contenant 5 g de bismuth.

Après 4 heures de contact, les pastilles sont séchées à 120°C pendant 24 heures.

Elles sont ensuite imprégnées par une solution d'acide chloroplatinique contenant 5 grammes de platine.

Après 4 heures de contact, les pastilles sont séchées à 120°C puis activées pendant 3 heures à 300°C dans un courant d'hydrogène circulant à 200 litres par heure.

Le catalyseur (B) ainsi préparé contient en poids rapporté au support de charbon actif 5 % de platine et 5% de bismuth.

### EXEMPLE 3 : Préparation d'un catalyseur (C) : 5%Pt/5%Bi sur charbon actif

100 grammes de charbon actif commercialisé par la société CECA sous l'appellation CECA 3S sont lavés successivement par une solution d'acide chlorhydrique puis par de l'eau permutée pour éliminer les impuretés solubles. Le support est ensuite séché à l'étuve à 120°C pendant 24 heures.

Ces pastilles sont ensuite imprégnées par une solution d'acide hexachoroplatinique contenant 5 g de platine.

Après 4 heures de contact, les pastilles sont séchées à 120°C pendant 24 heures.

Elles sont ensuite imprégnées par une solution de nitrate de bismuth contenant 5 grammes de bismuth.

Après 4 heures de contact, les pastilles sont séchées à 120°C puis activées pendant 3 heures à 300°C dans un courant d'hydrogène circulant à 200 litres par heure.

Le catalyseur (C) ainsi préparé contient en poids rapporté au support de charbon actif 5% de bismuth et 5% de platine.

### EXEMPLE 4 : Préparation d'un catalyseur (D) : 5%Bi/4%Pd/1%Pt sur charbon actif

100 grammes de charbon actif commercialisé par la société CECA sous l'appellation CECA 3S sont lavés successivement par une solution d'acide chlorhydrique puis par de l'eau permutée pour éliminer les impuretés solubles. Le support est ensuite séché à l'étuve à 120°C pendant 24 heures.

Ces pastilles sont ensuite imprégnées par une solution de nitrate de bismuth contenant 5 g de bismuth.

Après 4 heures de contact, les pastilles sont séchées à 120 °C pendant 24 heures.

Elles sont ensuite imprégnées par une solution de nitrate de palladium et d'acide chloroplatinique contenant 4 grammes de palladium et 1 gramme platine.

Après 4 heures de contact, les pastilles sont séchées à 120°C puis activées pendant 3 heures à 300°C dans un courant d'hydrogène circulant à 200 litres par heure.

Le catalyseur (D) ainsi préparé contient en poids rapporté au support de charbon actif 4 % de palladium,1 % de platine et 5% de bismuth

### EXEMPLE 5 : Préparation d'un catalyseur (E) : 1%Bi/5%Pt sur carbone graphite

100 grammes de carbone graphite commercialisé par la société SN2A sous l'appellation Y 200 (noir d'acétylène) sont utilisés tel quels pour synthétiser le catalyseur (E). Le support est préalablement séché à l'étuve à 120°C pendant 24 heures.

Ce support en poudre est ensuite imprégné par une solution de nitrate de bismuth contenant 1 g de bismuth.

Après 4 heures de contact, le support imprégné est séché à 120°C pendant 24 heures.

Le catalyseur en poudre est ensuite imprégné par une solution de d'acide chloroplatinique contenant 5 grammes platine.

Après 4 heures de contact, le catalyseur est séché à 120°C puis activé pendant 3 heures à 300°C dans un courant d'hydrogène circulant à 20 litres par heure.

Le catalyseur (E) ainsi préparé contient en poids rapporté au support en graphite 5% de platine et 1 % de bismuth.

### EXEMPLE 6 : Oxydation du but-3-ène-1,2-diol (II) en acide 2-oxo-but-3-ènoïque (III) en présence du catalyseur (D)

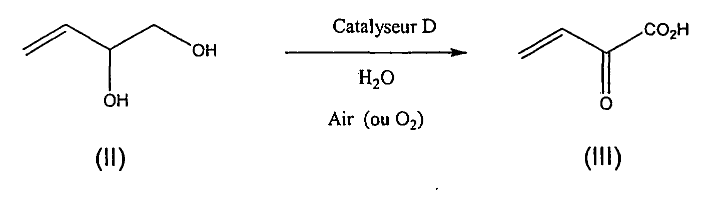

Les oxydations de solutions aqueuses à 0.1 à 50 % p/p de but-3-ène-1,2-diol (II) sont effectuées dans un réacteur en verre à double enveloppe d'une capacité de 500 mL, parfaitement agité. L'air est introduit par un tube plongeant munit optionnellement d'un fritté dans le milieu réactionnel. Le pH est régulé par ajout contrôlé de soude diluée au moyen d'une pompe asservie à un pH-mètre. Le pH, la consommation de soude, la température (50°C), et la pression partielle d'oxygène dans le ciel gazeux (à l'aide d'un oxymètre) sont enregistrés en continu.

Des prélèvements réguliers du milieu réactionnel sont opérés et les produits de la réaction sont analysés par chromatographie liquide haute pression (CLHP) et chromatographie en phase gazeuse (CPG).

Le catalyseur préalablement réduit et l'eau sont introduits dans le réacteur et la suspension est chauffée, sous agitation, à la température de réaction désirée sous un courant d'azote de manière à chasser l'oxygène dissous. Le but-3-ène-1,2-diol (II) est introduit et, au temps zéro, l'azote est remplacé par de l'air, le pH est ajusté à la valeur souhaitée et l'oxydation est amorcée.

Le taux de conversion du but-3-ène-1,2-diol est défini comme le pourcentage de but-3-ène-1,2-diol consommé. Le rendement en un produit est le pourcentage de but-3-ène-1,2-diol transformé en ce produit. La cinétique de réaction peut être suivie par la :
- disparition du but-3-ène-1,2-diol (II)
- quantité de soude nécessaire pour maintenir le pH constant du fait de la formation de l'acide (III).

Cet exemple est conduit en présence du catalyseur (D) obtenu à l'exemple 4, en opérant pendant une durée de 12 heures en atmosphère d'oxygène appauvrie et contrôlée.

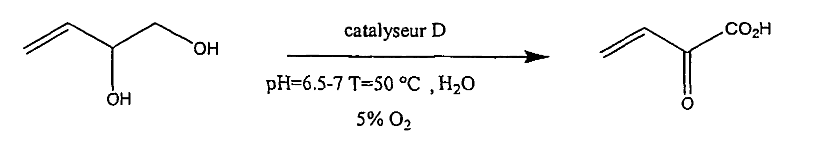

Dans un réacteur double enveloppe de 500 mL préalablement inerté à l'azote et équipé d'une agitation mécanique, d'une sonde de température et d'une sonde pH sont chargés :
Masse de catalyseur introduite : 0,302 g

| Réactifs | Pureté | Masse molaire (g/mole) | Masse introduite (g) | Nombre de moles | Nombre d'équivalent |
|---|---|---|---|---|---|
| 3-butène-1,2-diol | 99% | 88,11 | 4,1093 | 0,05 | 1,00 |
| Eau | 100% | 18 | 300 | 16,67 | 361 |

Le milieu réactionnel est porté à 50°C. Le débit d'air est fixé à 2,6 L/h celui d'azote à 8 L/h. Le %O₂ mesuré via un oxymètre affiche 5% vol. environ. La vitesse d'agitation est fixée à 300 tours/min. Le pH du milieu réactionnel est régulé dans la plage 6-7 par adjonction de soude aqueuse diluée (0.15% p/p). La vitesse de disparition du but-3-ène-1,2-diol est mesurée par chromatographie en phase gazeuse. L'apparition du α-céto-acide (III) est déterminée par chromatographie liquide haute pression. Les résultats sont :
- Taux Transformation (diol II) = 38% après 6 heures
- Taux Transformation (diol II) = 64% après 12 heures

### EXEMPLE 7 : Oxydation du but-3-ène-1,2-diol (II) en acide 2-oxo-but-3-ènoïque (III) en présence du catalyseur (D)

Cet exemple est conduit en présence du catalyseur (D) obtenu à l'exemple 4, en opérant pendant une durée de 4 heures en présence d'air à pH = 7,5.

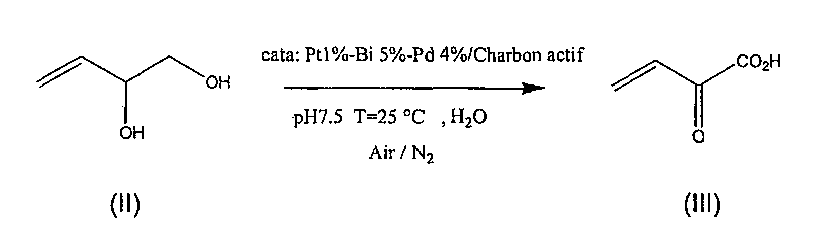

Dans un réacteur double enveloppe de 100 mL préalablement inerté à l'azote et équipé d'une agitation mécanique, d'une sonde de température et d'une sonde pH sont chargés :
Catalyseur (D): 0,5 g
Diol (II) : 0,5 g

Le milieu réactionnel est maintenu à 30°C. Le débit d'air est fixé à 12 L/h celui d'azote à 9 L/h. La vitesse d'agitation est fixée à 1300 tours/min. Le pH du milieu réactionnel est régulé à 7,5 par adjonction de soude aqueuse diluée (0,5% p/p). La vitesse de disparition du but-3-ène-1,2-diol (II) est mesurée par chromatographie en phase gazeuse. L'apparition de l'acide-2-oxo-but-3-ènoïque (III) est déterminée par chromatographie liquide haute pression.

Les performances mesurées sont :
- Taux Transformation (diol II) = 75% après 1 h
- Taux Transformation (diol II) >99% après 4,5 h
- Rendement en acide-2-oxo-but-3-ènoïque (III) > 85%

### EXEMPLE 8 : Illustration de la valeur nutritionnelle de l'acide 2-oxo-4-méthylthiobutyrique (KMB) chez le poulet, comme source de méthionine

### 8.1) Principe expérimental :

On a utilisé un modèle expérimental dit en dose réponse.

On part d'une base aliment carencée en méthionine, la méthionine étant l'élément nutritionnel pour lequel on veut observer une réponse, puis on introduit ledit élément nutritionnel manquant sous la forme d'acide 2-hydrox-4-méthylthiobutyrique (produit fabriqué par la Demanderesse sous le nom de Rhodimet^{™} AT88) selon le régime R2 et sous la forme d'acide 2-oxo-4-méthylthiobutyrique (KMB) selon le régime R3, aux mêmes doses, puis on compare les performances obtenues dans chacun des régimes, le régime R1 correspondant à l'absence d'introduction de méthionine.

### 8.2) Schéma expérimental :

Les poulets testés sont au nombre de 4 par cage.

Trois traitements R1 (sans méthionine ajoutée), R2 (+ Rhodimet^{™} AT88) et R3 (+ KMB) ont été réalisés, qui sont répétés neuf fois chacun, selon le tableau 1 suivant.

**Tableau 1**

| | | | |
|---|---|---|---|
| Base aliment sur 0-7 jours | 0,45% Met totale | | |
| Base aliment sur 7-21 jours | Maïs soja croissance 0,32% Met totale | | |
| Régime | R1 | R2 | R3 |
| Item | -------- | Rhodimet^{™} AT88 | KMB |
| Dose item (%) | -------- | 0,09 | 0,09 |
| Effectifs | 36 | 36 | 36 |

Le tableau 2 ci-après présente les résultats obtenus sur la période de 7-21 jours.

**Tableau 2**

| | Régimes Item Dose Met (%) | R1 ----- 0 | R2 Rhodimet^{™} AT88 0,09 | R3 KMB 0,09 |
|---|---|---|---|---|
| Poids indiv | Moyennes | 134,8 | 135,1 | 134,9 |
| J7 | Ecart type | 1,85 | 0,93 | 1,82 |
| (g) | CV | 1,37 36 | 0,69 36 | 1,35 36 |
| | Nb animaux | | | |
| Poids indiv | Moyennes | 641 a | 744 b | 730 b |
| J21 | Ecart type | 56,6 | 28,0 | 28,5 |
| (g) | CV | 8,84 | 3,76 | 3,90 |
| | Nb animaux | 36 | 36 | 36 |
| | Delta (%) | | 16,1 | 14,0 |
| Gain de poids | Moyennes | 506 a | 609 b | 596 b |
| J7-J21 | Ecart type | 55,8 | 27,9 | 27,1 |
| (g) | CV | 11,02 | 4,58 | 4,54 |
| | Delta (%) | | 20,4 | 17,7 |
| Ingéré | Moyennes | 971a | 1036 b | 1044 b |
| J7-J21 | Ecart type | 80,3 | 40,3 | 68,2 |
| | CV | 8,27 | 3,89 | 6,54 |
| (g) | Delta (%) | | 6,7 | 7,5 |
| Indice de Consommation | Moyennes | 1,930 a | 1 ,702 b | 1,752 b |
| J7-J21 | Ecart type | 0,1633 | 0,0423 | 0,0836 |
| (g/g) | CV | 8,46 | 2,49 | 4,77 |
| | Delta (%) | | -11,8 | -9,2 |

| | | | | |
|---|---|---|---|---|
| NB : Les valeurs affectées d'une lettre a ou b sont significativement différentes au seuil de 5% | | | | |

L'indice de consommation sur une période donnée est le ratio aliment ingéré sur gain de poids, sur cette même période, ce qui correspond à la quantité d'aliment nécessaire pour obtenir un gain de poids de 1 kg.

L'apport en méthionine sous forme de KMB (R3) permet une amélioration du gain poids sur la période d'environ 90g et une diminution de l'indice de consommation de 9% non significativement différentes de celles obtenues avec l'hydroxy-analogue de méthionine (Rhodimet^{™} AT88, R2).

Le KMB a donc une valeur nutritionnelle équivalente de celle de l'hydroxy-analogue de méthionine.

### EXEMPLE 9 : Autre illustration de la valeur nutritionnelle de l'acide 2-oxo-4-méthylthiobutyrique (KMB) chez le poulet, comme source de méthionine

### 9.1) Principe expérimental :

On part d'une base aliment carencée en méthionine, la méthionine étant l'élément nutritionnel pour lequel on veut observer une réponse, puis on introduit ledit élément nutritionnel manquant sous les formes de référence et la forme à tester, puis on compare les performances obtenues dans chacun des régimes, le régime R1 correspondant à l'absence d'introduction de méthionine.

Les poulets testés sont au nombre de 2 par cage, en randomisation aléatoire contrôlée.

Sept traitements ont été réalisés :
R1 : sans méthionine ajoutée,
R2 et R3 : + Rhodimet^{™} NP99 (D,L-méthionine poudre commercialisée par la Demanderesse) à deux doses différentes
R4 et R5 : + Rhodimet^{™} AT88, à deux doses différentes
R6 et R7 : + KMB, à deux doses différentes.

Ils ont été répétés 14 fois chacun et sont représentés dans le tableau 3 suivant.

### Tableau 3

Les résultats obtenus sont présentés dans le tableau 4. Les résultats obtenus montrent que l'addition de 0,1% de méthionine sous forme NP99 permet d'augmenter significativement le gain de poids (+90g) et de diminuer significativement l'indice de consommation (-11%). L'absence d'effet de la dose de supplémentation suggère que les besoins en méthionine étaient couverts dès la dose de 0,1%.

Les résultats obtenus avec l'ajout d'AT88 ou de KMB sont similaires et non significativement différents de ceux obtenus avec la DL-méthionine (NP99). Le KMB a donc comme l'AT88 une valeur nutritionnelle équivalente à celle de la DL-méthionine.

| | | | | | | | |
|---|---|---|---|---|---|---|---|
| Base aliment 0-7 jours | Maïs soja starter (50%NP99 et 50% AT88, pour un équivalent de 0,22% de Met ajoutée) | | | | | | |
| Base aliment 7-21 jours | Maïs soja croissance | | | | | | |
| Régimes | R1 | R2 | R3 | R4 | R5 | R6 | R7 |
| Item | -- | NP 99 | NP 99 | AT88 | AT88 | KMB | KMB |
| Doses item (%) | -- | 0.100 | 0.200 | 0.113 | 0.225 | 0.102 | 0.204 |
| Poids d'aliment farine à traiter (Kg) | 50 | 50 | 50 | 50 | 50 | 50 | 50 |
| Poids d'aliment expérimental Granulés (Kg) | 40 | 40 | 40 | 40 | 40 | 40 | 40 |
| Effectif en expérience | 28 | 28 | 28 | 28 | 28 | 28 | 28 |

**Tableau 4**

| Performances zootechniques en fonction des traitements (période 7-21 jours) | | | | | | | | |
|---|---|---|---|---|---|---|---|---|
| | Régimes Item Dose | R1 Témoin / | R2 NP99 0,100% | R3 NP99 0,200% | R4 AT88 0,113% | R5 AT88 0,225% | R6 KMB 0,102% | R7 KMB 0,204% |
| Mortalité | J7-J21 | 3,6% | 0,0% | 0,0% | 0,0% | 0,0% | 7,1% | 0,0% |
| Gain de poids | Moyennes | 454 a | 544 b | 527 b | 549 b | 559 b | 563 b | 535 b |
| J7-J21 | Ecart type | 56,4 | 73,3 | 78,6 | 51,2 | 49,0 | 57,4 | 44,0 |
| | CV | 12,42 | 13,48 | 14,90 | 9,31 | 8,76 | 10,18 | 8,23 |
| (g) | Delta (%) | | 19,8 | 16,2 | 21,0 | 23,2 | 24,1 | 17,9 |
| Conso | Moyennes | 780 | 832 | 823 | 850 | 846 | 864 | 819 |
| J7-J21 | Ecart type | 98,9 | 113,5 | 116,5 | 64,9 | 65,2 | 65,4 | 67,6 |
| | CV | 12,67 | 13,64 | 14,15 | 7,64 | 7,70 | 7,58 | 8,25 |
| (g) | Delta (%) | | 6,7 | 5,5 | 9,0 | 8,4 | 10,7 | 5,0 |
| IC | Moyennes | 1,722 a | 1,531 b | 1,564 b | 1,552 b | 1,514 b | 1,539 b | 1,533 b |
| J7-J21 | Ecart type | 0,1271 | 0,0562 | 0,0653 | 0,0829 | 0,0653 | 0,0895 | 0,0731 |
| | CV | 7,38 | 3,67 | 4,18 | 5,34 | 4,31 | 5,81 | 4,77 |
| | Nb valeurs | 13 | 14 | 14 | 14 | 14 | 12 | 14 |
| | Delta (%) | | -11,1 | -9,2 | -9,9 | -12,1 | -10,6 | -11,0 |

### Valeur biologique du KMB :

Les résultats obtenus selon le tableau 4 permettent également de calculer une valeur biologique « approximative » du KMB. La courbe dose réponse n'étant basée que sur deux points, cette valeur ne peut être considérée comme définitive mais permet d'avoir une approche plus quantitative quant à la valeur biologique du KMB sans préjuger de sa valeur d'usage.

Pour comparer les deux produits, on utilise le calcul d'efficacité de transformation de la méthionine qui consiste à déterminer la quantité moyenne de matière active (méthionine ou KMB en mg) nécessaire pour obtenir les grammes de gain de poids supplémentaires par rapport au gain de poids du témoin carencé. Plus le nombre de grammes nécessaires à l'obtention d'un gramme de gain de poids est faible, plus l'efficacité du produit est élevée, comme l'illustre la figure.

Le rapport d'efficacité du KMB par rapport à la DL-méthionine est alors calculé en faisant le rapport des valeurs d'efficacité de transformation calculées aux deux doses utilisées. Ainsi les valeurs d'efficacité relative montrent que le KMB présente une meilleure efficacité que la DL-méthionine, respectivement 118,5 et 111,5 % pour les doses 0,1 et 0,2 %.

En conclusion, les exemples 8 et 9 démontrent que le KMB apporté dans l'aliment est assimilé par l'animal au niveau intestinal et qu'il est utilisé comme une source de méthionine de manière au moins équivalente à la DL-méthionine pour la croissance.

## Revendications

1. Procédé pour préparer l'acide 2-oxo-4-méthylthiobutyrique et
ses sels, ledit acide répondant à la formule (I) 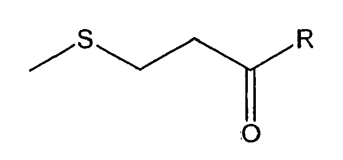 dans laquelle R représente un groupement carboxylique, et ses sels, ledit procédé étant **caractérisé en ce qu'**il comprend les étapes suivantes :
on effectue l'oxydation catalytique et sélective du but-3-ène-1,2-diol (II) en acide-2-oxo-but-3-ènoïque (III) selon le schéma réactionnel (i) suivant : 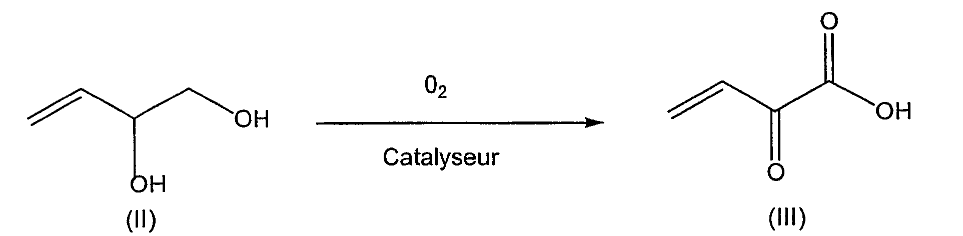
et on condense sélectivement le méthylmercaptan sur l'acide-2-oxo-but-3-ènoïque (III) selon le schéma réactionnel (ii) suivant :

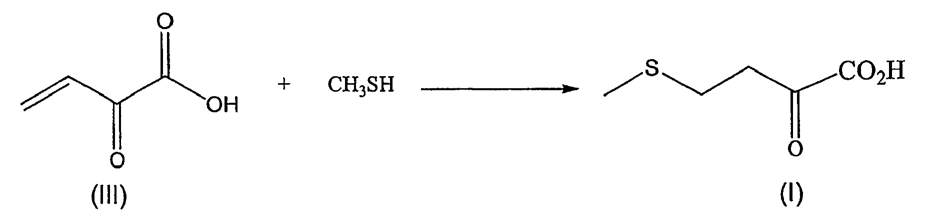

2. Procédé pour préparer un composé répondant à la formule (I) 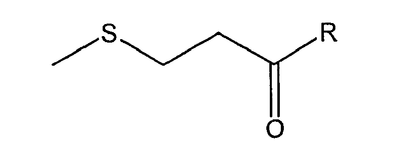 dans laquelle R représente un groupement choisi parmi COOR', CONH₂, CONHR', CONR'R" où R' et R" sont choisis, indépendamment l'un de l'autre, dans le groupe des radicaux alkyles linéaires ou ramifiés ayant de 1 à 12 atomes de carbone, et des radicaux cycloalkyles ayant de 3 à 12 atomes de carbone,
**caractérisé en ce qu'**on obtient l'acide 2-oxo-4-méthylthiobutyrique (I) selon le procédé défini à la revendication 1 puis on effectue une étape d'estérification ou d'amidification.

3. Procédé selon la revendication 1 ou 2, **caractérisé en ce que** le catalyseur comprend au moins un métal noble choisi parmi le palladium, le platine, le ruthénium, l'iridium, le rhodium et leurs mélanges.

4. Procédé selon la revendication 3, **caractérisé en ce que** le catalyseur comprend au moins un promoteur choisi parmi le bismuth, le plomb, l'antimoine, l'étain, le niobium, le tellure, l'indium, le gallium, le zinc, le cuivre, le nickel, le cobalt, l'or, l'argent, le tungstène, le molybdène, le rhénium, le vanadium, le chrome, le manganèse, le fer et leurs mélanges.

5. Procédé selon la revendication 3 ou 4, **caractérisé en ce que** le catalyseur comprend un support inerte choisi parmi l'alumine, la silice, les charbons actifs, le graphite, l'oxyde de titane, le zircone, le carbure de silicium, les oxydes mixtes à base de zirconium et de cérium, le noir d'acétylène.

6. Procédé selon les revendications 4 et 5, **caractérisé en ce que** le promoteur est déposé sur le support par imprégnation.

7. Procédé selon l'une quelconque des revendications 3 à 6, **caractérisé en ce que** le catalyseur comprend un métal noble choisi parmi le palladium, le platine et leurs mélanges, un promoteur choisi parmi le bismuth, le plomb et leurs mélanges et un support choisi parmi le charbon actif et le graphite.

8. Procédé selon l'une quelconque des revendications 3 à 7, **caractérisé en ce que** la teneur du métal ou des métaux nobles est comprise entre 0,1 et 10% en poids par rapport au support catalytique, et de préférence, comprise entre 0,5 et 5% en poids.

9. Procédé selon l'une quelconque des revendications 4 à 8, **caractérisé en ce que** la teneur du promoteur est comprise entre 0,005 et 500%, de préférence entre 0,005 et 100%, en poids du poids du métal ou des métaux nobles.

10. Procédé selon l'une quelconque des revendications 4 à 9, **caractérisé en ce que** la teneur du promoteur peut atteindre 10% en poids du poids du catalyseur.

11. Procédé selon l'une quelconque des revendications 3 à 10, **caractérisé en ce qu'**on effectue l'oxydation en milieu neutre ou alcalin, à un pH maintenu entre 4 et 11, de préférence entre 5,5 et 7,5.

12. Procédé selon la revendication 11, **caractérisé en ce qu'**on ajoute un agent alcalin choisi parmi l'hydroxyde de calcium, l'hydroxyde de sodium, l'hydroxyde de potassium, l'hydroxyde de lithium, l'hydroxyde de magnésium, l'ammoniac, le carbonate de sodium, le carbonate de zinc, le carbonate de manganèse et leurs mélanges.

13. Procédé selon l'une quelconque des revendications 1 à 12, **caractérisé en ce qu'**on amorce l'oxydation par l'apport d'un balayage d'un gaz contenant de l'oxygène, comme de l'air.

14. Procédé selon l'une quelconque des revendications 1 à 13, **caractérisé en ce que** l'oxydation est réalisée à une température comprise entre 10 et 95°C, de préférence entre 20 et 95°C, voire entre 25 et 70°C.

15. Procédé selon l'une quelconque des revendications 1 à 14, **caractérisé en ce que** l'oxydation se déroule sur une période comprise entre 20 minutes et 15 heures.

16. Procédé selon l'une quelconque des revendications 1 à 15, **caractérisé en ce que** pour condenser le méthylmercaptan sur l'acide-2-oxo-but-3-ènoïque, on utilise le méthylmercaptan sous forme gazeuse ou sous forme liquide.

17. Procédé selon la revendication 16, **caractérisé en ce que** la condensation est effectuée en présence d'un catalyseur basique.

18. Procédé selon la revendication 17, **caractérisé en ce que** le catalyseur est choisi parmi les amines aliphatiques telles que la méthylamine, l'éthylamine, la propylamine, la butylamine, la pentylamine, l'hexylamine, l'heptylamine et l'octylamine, les amines aromatiques comme l'aniline, la pyridine, l'hexaméthylènetétramine, la triéthylamine, la diisopropyléthylamine, diazabicylo[2,2,2]octane, la N,N-diméthylbenzylamine, la N-méthyldiphénylamine, la N-éthyl-3,3'-diphényldipropylamine, une N-alkylmorpholine, telle que la N-méthylmorpholine, ou bien le triton B.

19. Procédé selon la revendication 18, **caractérisé en ce que** le catalyseur est une N-alkylmorpholine, telle que la N-méthylmorpholine, combinée à un acide organique choisi parmi l'acide formique, l'acide acétique, l'acide propanoïque et l'acide butanoïque.

20. Procédé pour préparer l'acide-2-oxo-but-3-énoïque (III), **caractérisé en ce qu'**on effectue l'oxydation catalytique et sélective du but-3-ène-1,2-diol (II), selon l'une quelconque des revendications 1 à 15.

## Claims

1. Process for preparing 2-oxo-4-methylthiobutyric acid and its salts, the said acid corresponding to the formula (I) 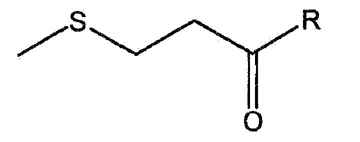 in which R represents a carboxyl group, and its salts, the said process being **characterized in that** it comprises the following stages:
but-3-ene-1,2-diol (II) is catalytically and selectively oxidized to give 2-oxobut-3-enoic acid (III), according to the following reaction scheme (i): 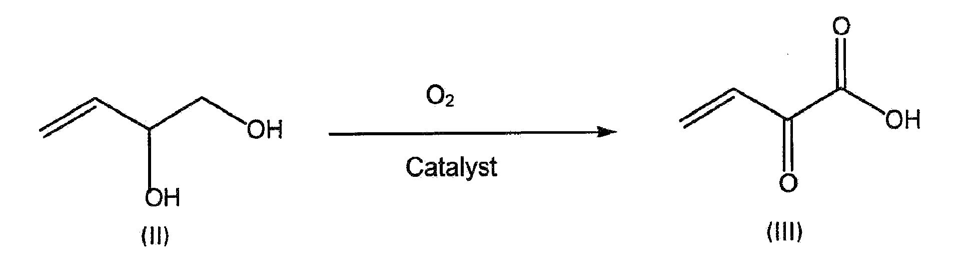
and methyl mercaptan is selectively condensed with 2-oxobut-3-enoic acid (III), according to the following reaction scheme (ii):

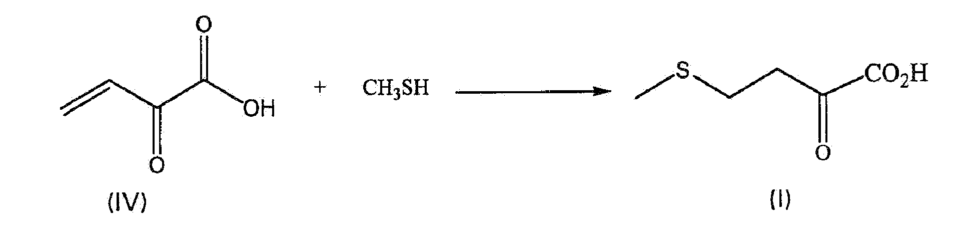

2. Process for preparing a compound corresponding to the formula 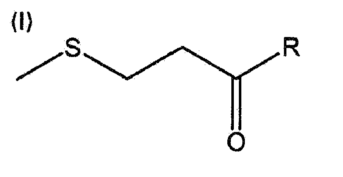 in which R represents a group chosen from COOR', CONH₂, CONHR' or CONR'R", where R' and R" are chosen, independently of one another, from the group of linear or branched alkyl radicals having from 1 to 12 carbon atoms and cycloalkyl radicals having from 3 to 12 carbon atoms,
**characterized in that** 2-oxo-4-methylthiobutyric acid (I) is obtained according to the process defined in claim 1 and then an esterification or amidation stage is carried out.

3. Process according to claim 1 or 2, **characterized in that** the catalyst comprises at least one noble metal chosen from palladium, platinum, ruthenium, iridium, rhodium and their mixtures.

4. Process according to claim 3, **characterized in that** the catalyst comprises at least one promoter chosen from bismuth, lead, antimony, tin, niobium, tellurium, indium, gallium, zinc, copper, nickel, cobalt, gold, silver, tungsten, molybdenum, rhenium, vanadium, chromium, manganese, iron and their mixtures.

5. Process according to claim 3 or 4, **characterized in that** the catalyst comprises an inert support chosen from alumina, silica, active charcoals, graphite, titanium oxide, zirconia, silicon carbide, mixed oxides based on zirconium and on cerium, acetylene black.

6. Process according to claim 4 or 5, **characterized in that** the promoter is deposited on the support by impregnation.

7. Process according to any one of claims 3 to 6, **characterized in that** the catalyst comprises a noble metal chosen from palladium, platinum and their mixtures, a promoter chosen from bismuth and lead and their mixtures and a support chosen from active charcoal and graphite.

8. Process according to any one of claims 3 to 7, **characterized in that** the content of the noble metal or metals is between 0.1 and 10% by weight with respect to the catalytic support, and preferably between 0.5 and 5%.

9. Process according to any one of claims 4 to 8, **characterized in that** the content of the promoter is between 0.005 and 500% by weight of the weight of the noble metal or metals, and preferably between 0.005 and 100%.

10. Process according to any one of claims 4 to 9, **characterized in that** the content of the promoter can reach 10% by weight of the weight of the catalyst.

11. Process according to any one of claims 3 to 10, **characterized in that** the oxidation is carried out in a neutral or alkaline medium, at a pH maintained between 4 and 11, preferably between 5.5 and 7.5.

12. Process according to claim 11, **characterized in that** an alkaline agent chosen from calcium hydroxide, sodium hydroxide, potassium hydroxide, lithium hydroxide, magnesium hydroxide, ammonia, sodium carbonate, zinc carbonate, manganese carbonate and their mixtures is added.

13. Process according to any one of claims 1 to 12, **characterized in that** the oxidation is initiated by starting to flush with a gas comprising oxygen, such as air.

14. Process according to any one of claims 1 to 13, **characterized in that** the oxidation is carried out at a temperature of between 10 and 95°C, preferably between 20 and 95°C, and even more preferably between 25 and 70°C.

15. Process according to any one of claims 1 to 14, **characterized in that** the oxidation takes place over a period of between 20 minutes and 15 hours.

16. Process according to any one of claims 1 to 15, **characterized in that**, in order to condense methyl mercaptan with 2-oxobut-3-enoic acid, methyl mercaptan is used in the gaseous form or in the liquid form.

17. Process according to claim 16, **characterized in that** the condensation is carried out in the presence of a basic catalyst.

18. Process according to claim 17, **characterized in that** the catalyst is chosen from aliphatic amines, such as methylamine, ethylamine, propylamine, butylamine, pentylamine, hexylamine, heptylamine and octylamine, aromatic amines, such as aniline or pyridine, hexamethylenetetramine, triethylamine, diisopropylethylamine, diazabicylo-[2.2.2]octane, N,N-dimethylbenzylamine, N-methyldiphenylamine, N-ethyl-3,3'-diphenyldipropylamine or an N-alkylmorpholine, such as N-methylmorpholine, or triton B.

19. Process according to claim 18, **characterized in that** the catalyst is an N-alkylmorpholine, such as N-methylmorpholine, combined with an organic acid chosen from formic acid, acetic acid, propanoic acid and butanoic acid.

20. Process for preparing 2-oxobut-3-enoic acid (III), **characterized in that** but-3-ene-1,2-diol (II) is catalytically and selectively oxidized according to any one of claims 1 to 15.

## Patentansprüche

1. Verfahren zur Herstellung von 2-Oxo-4-methylthiobuttersäure und ihren Salzen, wobei die Säure der Formel (I) entspricht: 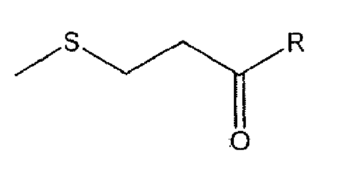 worin R für eine Carboxylgruppe steht, und ihren Salzen, wobei das Verfahren **dadurch gekennzeichnet ist, daß** man:
But-3-en-1,2-diol (II) gemäß dem folgenden Reaktionsschema (i) katalytisch und selektiv zu 2-Oxo-but-3-ensäure (III) oxidiert: 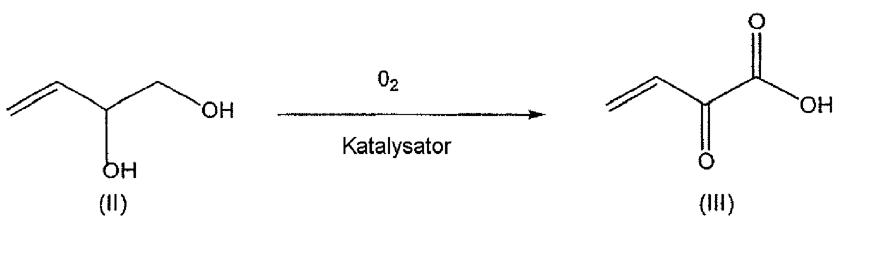
und 2-Oxo-but-3-ensäure (III) gemäß dem folgenden Reaktionsschema (ii) selektiv mit Methylmercaptan kondensiert:

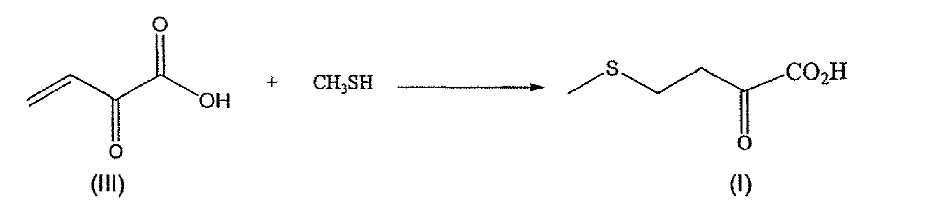

2. Verfahren zur Herstellung einer Verbindung der Formel (I), 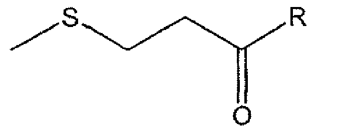 worin R für eine unter COOR', CONH₂, CONHR', CONR'R", worin R' und R" unabhängig voneinander aus der Gruppe der linearen oder verzweigten Alkylreste mit 1 bis 12 Kohlenstoffatomen und der Cycloalkylreste mit 3 bis 12 Kohlenstoffatomen ausgewählt sind, ausgewählte Gruppe steht, **dadurch gekennzeichnet, daß** man 2-Oxo-4-methylthiobuttersäure (I) gemäß dem in Anspruch 1 definierten Verfahren erhält und dann einen Veresterungs- oder Amidierungsschritt durchführt.

3. Verfahren nach Anspruch 1 oder 2, **dadurch gekennzeichnet, daß** der Katalysator mindestens ein unter Palladium, Platin, Ruthenium, Iridium, Rhodium und Mischungen davon ausgewähltes Edelmetall umfaßt.

4. Verfahren nach Anspruch 3, **dadurch gekennzeichnet, daß** der Katalysator mindestens einen unter Bismut, Blei, Antimon, Zinn, Niob, Tellur, Indium, Gallium, Zink, Kupfer, Nickel, Cobalt, Gold, Silber, Wolfram, Molybdän, Rhenium, Vanadium, Chrom, Mangan, Eisen und Mischungen davon ausgewählten Promotor umfaßt.

5. Verfahren nach Anspruch 3 oder 4, **dadurch gekennzeichnet, daß** der Katalysator einen inerten unter Aluminiumoxid, Siliciumdioxid, Aktivkohlen, Graphit, Titanoxid, Zirconiumoxid, Siliciumcarbid, Mischoxiden auf Basis von Zirconium und Cer und Acetylenruß ausgewählten Träger umfaßt.

6. Verfahren nach den Ansprüchen 4 und 5, **dadurch gekennzeichnet, daß** der Promotor durch Tränken auf dem Träger abgeschieden wird.

7. Verfahren nach einem der Ansprüche 3 bis 6, **dadurch gekennzeichnet, daß** der Katalysator ein unter Palladium, Platin und Mischungen davon ausgewähltes Edelmetall, einen unter Bismut, Blei und Mischungen davon ausgewählten Promotor und einen unter Aktivkohle und Graphit ausgewählten Träger umfaßt.

8. Verfahren nach einem der Ansprüche 3 bis 7, **dadurch gekennzeichnet, daß** der Metall- oder Edelmetallgehalt zwischen 0,1 und 10 Gew.-%, bezogen auf den Katalysatorträger, und vorzugsweise zwischen 0,5 und 5 Gew.-% liegt.

9. Verfahren nach einem der Ansprüche 4 bis 8, **dadurch gekennzeichnet, daß** der Promotorgehalt zwischen 0,005 und 500 Gew.-% und vorzugsweise zwischen 0,005 und 100 Gew.-%, bezogen auf das Metall- oder Edelmetallgewicht, liegt.

10. Verfahren nach einem der Ansprüche 4 bis 9, **dadurch gekennzeichnet, daß** der Promotorgehalt bis zu 10 Gew.-% des Katalysatorgewichts erreichen kann.

11. Verfahren nach einem der Ansprüche 3 bis 10, **dadurch gekennzeichnet, daß** man die Oxidation in neutralem oder alkalischem Medium bei einem zwischen 4 und 11 und vorzugsweise zwischen 5,5 und 7,5 gehaltenen pH-Wert durchführt.

12. Verfahren nach Anspruch 11, **dadurch gekennzeichnet, daß** man ein unter Calciumhydroxid, Natriumhydroxid, Kaliumhydroxid, Lithiumhydroxid, Magnesiumhydroxid, Ammoniak, Natriumcarbonat, Zinkcarbonat, Mangancarbonat und Mischungen davon ausgewähltes alkalisches Mittel zugibt.

13. Verfahren nach einem der Ansprüche 1 bis 12, **dadurch gekennzeichnet, daß** man die Oxidation beginnt, indem man mit einem sauerstoffhaltigen Gas wie Luft zu spülen beginnt.

14. Verfahren nach einem der Ansprüche 1 bis 13, **dadurch gekennzeichnet, daß** man die Oxidation bei einer Temperatur zwischen 10 und 95°C, vorzugsweise zwischen 20 und 95°C oder auch zwischen 25 und 70°C durchführt.

15. Verfahren nach einem der Ansprüche 1 bis 14, **dadurch gekennzeichnet, daß** die Oxidation über einen Zeitraum zwischen 20 Minuten und 15 Stunden erfolgt.

16. Verfahren nach einem der Ansprüche 1 bis 15, **dadurch gekennzeichnet, daß** man zur Kondensation der 2-Oxobut-3-ensäure mit dem Methylmercaptan das Methylmercaptan in gasförmiger oder flüssiger Form verwendet.

17. Verfahren nach Anspruch 16, **dadurch gekennzeichnet, daß** man die Kondensation in Gegenwart eines basischen Katalysators durchführt.

18. Verfahren nach Anspruch 17, **dadurch gekennzeichnet, daß** man den Katalysator aus der Gruppe der aliphatischen Amine wie Methylamin, Ethylamin, Propylamin, Butylamin, Pentylamin, Hexylamin, Heptylamin und Octylamin, der aromatischen Amine wie Anilin, Pyridin, Hexamethylentetramin, Triethylamin, Diisopropylethylamin, Diazabicyclo-[2.2.2]octan, N,N-Dimethylbenzylamin, N-Methyldiphenylamin, N-Ethyl-3,3'diphenyldipropylamin, einem N-Alkylmorpholin wie N-Methylmorpholin oder auch Triton B auswählt.

19. Verfahren nach Anspruch 18, **dadurch gekennzeichnet, daß** es sich bei dem Katalysator um ein N-Alkylmorpholin wie N-Methylmorpholin in Kombination mit einer unter Ameisensäure, Essigsäure, Propansäure und Butansäure ausgewählten organischen Säure handelt.

20. Verfahren zur Herstellung von 2-Oxobut-3-ensäure (III), **dadurch gekennzeichnet, daß** man But-3-en-1,2-diol (II) nach einem der Ansprüche 1 bis 15 katalytisch und selektiv oxidiert.
